# EUROPEAN PATENT SPECIFICATION

(11) **EP 1 368 655 B1**
(45) Date of publication and mention of the grant of the patent: **21.03.2007**
(21) Application number: 01949652.0
(22) Date of filing: 11.07.2001
(51) Int. Cl.: G01N 33/542, G01N 33/58, G01N 33/68

(54) **DISTINGUISHING MOLECULAR FORMS**
UNTERSCHEIDUNG VON MOLEKULAREN FORMEN
DISTINGUER DES FORMES MOLECULAIRES

(30) Priority: 11.07.2000 GB 0016953
(43) Date of publication of application: 10.12.2003
(73) Proprietor: Microsens Biophage Limited, London NW1 0TU (GB)
(72) Inventor: WILSON, Stuart Mark, London SE25 6TW (GB); STANLEY, Christopher, Ivotech, Harradine House, Huntingdon, Cambs PE28 3BN (GB)
(74) Representative: Smart, Peter John
(86) International application number: PCT/GB2001/003074
(87) International publication number: WO 2002/004948

(56) References cited:
- EP-A- 0 439 354
- WO-A-01/23894
- WO-A-99/63348
- US-A- 5 849 878
- Bennion; Daggett (2002) Clinical Chemistry 48, 2105-2114.

## Description

### FIELD OF THE INVENTION

The present invention relates to a method for distinguishing aggregated or polymerized forms of a given molecule from the unaggregated or unpolymerized form of the same molecule or a closely similar molecule.

### BACKGROUND TO THE INVENTION

Methods exist to detect the interaction between two different molecules. A dual bacteriophage method has been described in WO 99/63348 in which each molecule for which the interaction must be detected is labelled by a bacteriophage encoding a different selectable marker. As the molecules interact the phage are brought into proximity and can infect the same host bacilli thereby conferring the double selectable marker phenotype on the host. A yeast hybrid method has been described in which the proteins of interest are expressed in the yeast genome. If the proteins interact they activate a promoter which generates a measurable signal. A method called Fluorescence Excitation Transfer (FET) exists in which a transfer of energy can be measured between two ligands that are used to label each interacting molecule and are brought into close proximity by the interaction of these molecules (for a review of possible labels and approaches see Wood, P and Barnard, G. 1997. Fluoroimmunoassay in Principles and Practice of Immunoassay. Price and Newman Eds. Macmillan Reference Ltd., UK).

In some cases, however, it is desirable to measure or detect a change in a given molecule that causes the molecule to bind together or aggregate or polymerise. For example, in some diseases such as the transmissible spongiform encephalopathies (TSEs) there has been a change in the normal cellular protein PrPc which is not aggregated to a form such as PrPsc which is aggregated or bound together. This aggregation and subsequent deposition in vivo may cause the pathology of these diseases. A considerable problem in diagnosis or detection of such diseases is the similarity of the normal and pathogenic proteins. Methods must be devised to differentiate the normal from the pathogenic form. It is difficult to raise antibodies, for example, which are specific for the pathogenic form and most antibodies cross-react with the normal cellular protein. Consequently, existing diagnostic methodologies must employ a complicated enrichment sample preparation procedure involving detergent extraction, protease treatment and/or solvent extraction in order to preferentially select the pathogenic protein prior to performing the assay.

WO 01/23894 A1 discloses a method for detecting aggregates of molecules by labelling with two fluorescent antibody probes, each having a different site specificity, and detecting aggregates intensely labelled with both probes.

The present invention seeks to provide an improved method of differentiating aggregated molecules from unaggregated normal molecules.

### DISCLOSURE OF THE INVENTION

According to an aspect of the present invention there is provided a method for detecting aggregation or polymerisation of molecules in a sample, which method comprises the steps of:
a) exposing a sample containing the molecules to at least two different labelling moieties each of which will bind to only a single site on each of the said molecules such that binding of one of said labelling moieties to a said site will exclude the binding to the same molecule of a different said labelling moiety; and
b) determining whether at least two different said labelling moieties are present linked together via aggregates or polymers of said molecules.

Preferably the labelling moieties respectively comprise a first virus and a second virus which are generally different from each other. Both viruses are capable of binding directly or indirectly to only a single site on each of the said molecules. In addition, the viruses bind directly or indirectly to the same site on the said molecule so that binding of one virus excludes the binding of the other virus. The viruses either bind to the same identical site or a similar overlapping site such that when one virus is bound the other virus is prevented from binding. The latter can be achieved by steric hindrance by the first-binding virus thus preventing the second virus from binding or conformational change in the target material thus preventing the second virus binding.

In a preferred embodiment of the method the virus bind indirectly to an identical or overlapping site through monoclonal antibodies that are specific for an identical or overlapping sites. Hereafter, this property of the binding of one labelling moiety to the target molecule excluding the binding of the second or other labelling moieties is termed 'mutually exclusive binding', and the term 'mutually exclusive site' refers to a site on a molecule on which mutually exclusive binding occurs. The binding of two or more labelling moieties to the target material forms a material that has a distinctive property when it includes both the first and the second virus linked together via aggregated or polymerized molecules.

The term "virus" is used herein to denote true viruses and organisms which infect bacteria in manner similar to a true virus. Thus, the term virus includes:
a. Components of a virus which have the characteristics of the virus from which they are derived;
b. Packaged phagemids, which are crosses between plasmids and viruses and can grow as plasmids in bacterial hosts, but which can be packaged and secreted as if they were viral particles in the presence of a helper virus although they cannot independently produce viral progeny;
c. Viruses which are lysogenic for bacteria and can grow, replicate and produce progeny in the bacteria without lysis of the bacteria which can continue to grow and replicate.

If viral infection of bacterial cells is carried out using an excess of bacteria over that required to achieve parity between the infecting viruses and the infectable bacterial cells, a given bacterium cell is unlikely to be infected by more than one virus particle. The amount at which such dual infection becomes sufficiently unlikely that it will not distort the results of the assay method of the invention can be calculated statistically and is denoted herein as the statistical amount. Such a statistical calculation can be confirmed by simple trial and error tests.

In a preferred embodiment of the invention, two viral particles are physically linked together through the target material and can thus each infect the same bacterial cell so as to endow that cell with both of the characteristic properties of the infecting viruses. A bacterial cell infected by both viruses and possessing the sum of the two characteristic properties can readily be distinguished from cells which possess only one of those properties. For example, the infected cells can be cultivated under conditions under which the cells possessing only one of the properties cannot survive, for example in the presence of specific antibiotics or specific temperature or pH conditions. The infected cells having both characteristic properties survive and will replicate. Thus, in the presence of tagged target material a cascade of bacterial growth indicates that the target material was present in the initial sample. If no tagged material is present in this cultivation stage, little or no bacterial growth will take place. In addition, if lysogenic viruses are used as the tags to be attached to the target material, the viruses will replicate within the infected bacterial cells and produce progeny virus particles which will be released to begin further cycles of infection and replication.

In a particularly preferred method of the invention a ligand such as a monoclonal antibody (mAbs) is used which recognises and binds to one site on the molecule of interest. In the unaggregated or non-pathogenic state only one ligand will bind to one molecule in isolation from all other ligands. However, when the molecule is in the pathogenic form and aggregated, a plurality of ligands bind to the aggregate through recognition of the binding sites on each individual molecule comprising the aggregate. In this case the aggregate contains many copies of the ligand. A single ligand can be differentiated from multiply bound ligands using various detection techniques. A proposition of the ligand can be labelled with a first moiety and a proportion of the ligand labelled with a second moiety such that in the aggregate with multiple ligand binding the energy transfer between the two moieties that have been brought into close proximity can be measured (for a review of possible labels and approaches see Wood, P and Barnard, G. 1997. Fluoroimmunoassay in Principles and Practice of Immunoassay. Price and Newman Eds. Macmillan Reference Ltd., (UK). In another example, the ligands could be labelled with the inactive components or subunits of a functional moiety such that when the components or subunits are brought together the moiety is endowed with a measurable function. In this example the subunits of an enzyme would be used as labels such that when brought together in proximity they partially restore or fully restore or alter the activity of the enzyme. It is preferred to use the double bacteriophage method described in WO 99/63348, with the two bacteriophage being labelled with the same ligand. In this example, when the two bacteriophage labelled with ligand are added to the assay the normal molecule will only bind one ligand and one phage so there will be no signal. However, an aggregate of the pathogenic molecules will bind many molecules of the ligand that will be labelled with either phage type. It is likely that a mixture of the two phage types will be bound to the aggregate through the ligand. The linking of the two phage types through the ligand and the aggregated pathogenic molecules generates a signal as described in WO 99/63348.

In a further embodiment of the invention a nucleic acid label may be used. In this case a ligand such as a monoclonal antibody is coupled to a single or double stranded nucleic acid molecule that acts as the label. Coupling can be undertaken by standard techniques, such as linking a 5' or a 3' amino DNA oligonucleotide to an antibody using bis succinimide or bis maleimide cross-linkers or glutaraldehyde. Short oliogonucleotides of 15 to 25 bases are preferred; longer DNA molecules of several hundred bases may also be employed. Under appropriate conditions the oligonucleotide coupled to one antibody via the 5' end is able to hybridise to the oligonucleotide coupled via the 3' end to another antibody molecule; this is preferably achieved using a prior cleavage with a restriction endonuclease that generates short complementary sequences ('sticky ends') at each end of the oligonucleotides prior to coupling to the antibodies.

When the monoclonal antibody labelled with either form of nucleic acid label binds to the aggregated form of a molecule the oligonucleotide labels will be juxtaposed and hybridization will be promoted. This will be a more favourable reaction than that taking place in either free solution (the background signal) or when binding to non-aggregated molecules. After hybridization of the oligonucleotides a ligation reaction, catalyzed either chemically or with DNA ligase can proceed; for single stranded DNA molecules a prior hybridization step is preferred with a complementary 'bridging' oligonucleotide that generates a double stranded DNA region as the substrate for the ligase. Blunt end ligation can also take place between two non-overlapping DNA oligonucleotide labels.

After ligation molecules of the type described by Cantor (US 5,849,878) are generated, that is two antibody molecules linked by a ligated DNA molecule. The product of ligation may then be detected by various means, such as hybridization of labelled probes across the ligated region, or by DNA amplification techniques such as PCR using primers situated either side of the ligation region. Other suitable DNA amplification techniques include RCA (rolling circle amplification) and LCR (ligase chain reaction).

### DESCRIPTION OF THE PREFERRED EMBODIMENT

The invention will now be described by way of illustration in terms of preferred embodiments thereof. In these examples the Dual Phage detection method is used to demonstrate the presence of polymerized bovine serum albumin (BSA) in the first example and polymerized tubulin in the second example. In the third example it is shown that the method can differentiate the aggregated PrP molecules associated with the Transmissible Spongiform Encephalopathy (TSE) disease scrapie from the normal unaggregated PrP molecules present in the normal non-diseased brain. The Dual Phage method uses two phagemid which encode two different antibiotic resistance genes: resistance to ampicillin in phage A and resistance to chloramphenicol in phage C. The phagemid are packaged into infectious particles using a M13-derived helper phage.

Method 1: In this method a model system was used to demonstrate that artificially cross-linked molecules could be differentiated from identical molecules that were not cross-linked.

### Preparation of the assay reagents

Anti-BSA monoclonal antibody (clone no. BSA-33, Sigma-Aldrich Company Ltd, UK) and the phage A and C were biotinylated using Biotinamidocaproic acid 3-sulfo-N-hydroxysuccinimide ester (Biotin ester) (Sigma-Aldrich Company Ltd, UK). The antibody was dialysed against PBS and 2.5 µl of biotin ester stock at 0.1 mg/ml in PBS added (this gives an average of approximately 1 biotin label per antibody molecule) and incubated at room temperature for 2 hours. Excess ester was removed by dialysis. Phage A and phage C were prepared following standard procedures (Phage Display of Peptides and Proteins: A Laboratory Manual. 1996. Kay, Winter and McCafferty, Eds. Academic Press, UK) and purified by caesium chloride density gradient centrifugation. After dialysis against PBS to remove the caesium chloride 10¹¹ plaque forming units of each phage were labelled in a volume of 100 µl with 10 µl of biotin ester (10 µg/ml in PBS)for 2 hours at room temperature. Excess ester was removed by polyethylene glycol precipitation of the phage following standard procedures (Phage Display of Peptides and Proteins: A Laboratory Manual. 1996. Kay, Winter and McCafferty, Eds. Academic Press, UK) and the phage pellet was resuspended in 100 µl PBS.

### Detection of cross-linked BSA

1. BSA, Fraction V (Sigma-Aldrich Company Ltd, UK) was cross-linked using serial dilutions of glutaraldehyde (Grade 1, 25% stock)(Sigma-Aldrich Company Ltd, UK). The BSA was prepared at 10 mg/ml in PBS and 100 µl aliquots cross-linked at room temperature for 2 hours with serial 5-fold dilutions of glutaraldehyde of final concentrations 2-0.0032% (v/v). A no-glutaraldehyde and a no-glutaraldehyde, no-BSA control were also included in the experiment.
2. After incubation the volume of each reaction was made up to 1 ml with TBS, 5 mM ethanolamine (Sigma-Aldrich Company Ltd, UK) and incubated for 2 hours at room temperature in order to quench the glutaraldehyde.
3. 1 µl of each reaction was added to 10 µl of 4% (w/v) sodium dodecylsulfate (Sigma-Aldrich Company Ltd, UK), 2.5% (v/v) B-mercaptoethanol (Sigma-Aldrich Company Ltd, UK)and heated at 95°C for 5 mins in order to denature the protein and expose the epitopes for antibody binding.
4. After denaturation, each reaction was made up to 1 ml with 50 mM sodium carbonate pH 9.6.
5. Each reaction was diluted a thousand-fold in the carbonate buffer and 100 µl of each was used to coat a maxisorb (Nunc, Denmark) microtiter plate well at 4°C overnight.
6. After coating, the wells were washed x3 with TBS 0.2% (v/v) Tween 20. The last wash was left in the well for 60 mins at room temperature.
7. The biotinylated anti-BSA monoclonal antibody was diluted 10⁻⁴ in TBS Tween20 and 100 µl added to each well.
8. After 1 hour at room temperature the wells were washed x3 with TBS Tween20 and 100 µl of streptavidin (Sigma-Aldrich Company Ltd, UK) (a stock solution of 1 mg/ml diluted 10⁻⁴ in TBS Tween20) added.
9. After 1 hour at room temperature the wells were washed x3 with TBS Tween20 and 100 µl of TBS Tween20 containing 10⁸ plaque forming units of each of the biotinylated phage C and phage A was added.
10. After incubation at room temperature for 1 hour the wells were washed x3 with TBS Tween20 and then x2 with TBS.
11. 1 ml of log phase growth XL-1 Blue (Stratagene) E. coli cells are then added to each well and the microtiter plate incubated at 37°C for 60 minutes.
12. The cell suspension from each well was then plated out on a 2xYT (Sigma Aldrich Chemical Company Ltd) 1.5% (w/v) agar containing 100 µg/ml ampicillin and 50 µg/ml chloramphenicol.
13. After incubation overnight at 37°C the number of colonies on each plate were counted.

### Results

| Percentage of glutaraldehyde used for cross-linking | Number of colonies on the plate |
|---|---|
| 2 | 3 |
| 0.4 | 66 |
| 0.08 | 50 |
| 0.016 | Greater than 1000 |
| 0.003 | 142 |
| 0 (no glutaraldehyde) | 70 |
| Control (no BSA) | 80 |

### Conclusion

This experiment demonstrates that the cross-linked BSA could be differentiated from the monomeric (uncrosslinked) BSA. The monoclonal antibody can only bind to one site on each BSA molecule. Consequently, this results in the capture of only one phage A or C to each molecule of BSA. This does not bring phage A and C into close proximity to allow the subsequent dual infection of the E. coli. Consequently, the E.coli remains uninfected or singly infected and does not grow on agar plates containing both antibiotics. However, when the BSA is cross-linked there are multiple molecules bound together in close-proximity each of which can bind one phage A or C. Thus phage A and phage C are brought into close proximity which allows dual infection of the E.coli that can then grow on the agar plates containing both antibiotics.

From the results, it can be seen that under these conditions and in this model system 0.016% (v/v) glutaraldehyde gave the highest signal. Lower concentrations of glutaraldehyde probably yielded few cross-linked molecules while at a higher concentration of glutaraldehyde the epitope detected by the antibody could be modified by the agent and destroyed preventing recognition by the antibody.

### Method 2.

In this method the aggregated form of a self-aggregating molecule was distinguished from the unaggregated form.
1. Anti-tubulin monoclonal antibody TUB 2.1 (Sigma Aldrich Company Ltd) was cross linked to each of the two bacteriophage, phage A and phage C, using standard conjunction chemistry protocols (Bioconjugation: Protein Coupling Techniques for the Biomedical Sciences. 1998. Aslam and Dent, Eds. Macmillan Reference Ltd., UK).
2. Two tubes containing 100 µl 2 mg/ml tubulin (T238, Cytoskeleton, Inc, Denver USA) were prepared. To one 10µl. of distilled water was added; this is the unpolymerized control tube. To the other tube 10µl of glycerol was added to initiate the polymerization process.
4. The tubes were incubated at 37°C for 30 mins.
5. Each reaction was diluted 10⁻⁶ and to 10µl of each dilution 10 µl of phage buffer (50 mM Na₃PO₄, 0.15 M NaCl, 1 mM MgCl₂ pH 7.2) containing 10⁵ plaque-forming units of anti-tubulin antibody-conjugated phage A and phage C was added and incubated 30 min at room temperature.
6. 1 ml of log phase growth XL-1 Blue (Stratagene) E. coli cells were then added to each tube and incubated at 37°C for 60 minutes.
7. The tubes were centrifuged for 5 minutes at 4000xg and the pellets resuspended in 50 µl of phage buffer.
8. The resuspended pellets were then plated out on two plates containing 2xYT media (Sigma Aldrich Chemical Company Ltd), 1.5% (w/v) agar and 100 µg/ml ampicillin and 50µg/ml chloramphenicol.
9. The plates were incubated overnight at 37°C and the resulting numbers of colonies of *E. coli* counted.

### Result:

The plate derived from the tube containing unpolymerized tubulin contained no colonies of *E. coli* growth. The plate derived from the tube of polymerized tubulin showed many colonies of *E. coli* growth (greater than 1000 colonies). The polymerized tubulin was able to link the two phage types such that they could infect the same bacterial host and confer resistance to both antibiotics which were subsequently used for selection in the agar plate.

This experiment shows that unaggregated or unpolymerized molecules, in this example of tubulin, can clearly be differentiated from the aggregated or polymerized form. The latter gives a clear positive signal when using a monoclonal antibody in the Dual Phage detection assay.

### Method 3

In this example material for the assay was prepared from scrapie-infected and uninfected ovine brain homogenates by detergent solubilization and low speed centrifugation clarification of debris as described in Saborio, G.P., Permanne, B., and Soto, C. 2001. Nature, 411: 810-813. No proteinase K step was performed.

To prepare the phage conjugates, the same anti-PrP monoclonal antibody was cross linked to each of the two bacteriophage, phage A and phage C, using standard conjunction chemistry protocols (Bioconjugation: Protein Coupling Techniques for the Biomedical Sciences. 1998. Aslam and Dent, Eds. Macmillan Reference Ltd., UK).
1. The wells of a Reacti-Bind Maleic Anhydride microtiter plate (Pierce, UK) were coated with 100 ng of an anti-PrP monoclonal antibody in carbonate buffer overnight at 4°C.
2. The wells were filled with 0.2 M ethanolamine pH 7.5 and incubated at room temperature for 2 hours.
3. 100 µl of the material extracted from the uninfected and the infected brain was added to separate wells and incubated at room temperature for 1 hour.
4. The wells were then washed x3 with PBS and then filled with 0.016% (v/v) glutaraldehyde in PBS.
5. After incubation for 2 hours at room temperature the wells were washed with PBS and then filled with 6 M urea.
6. After 30 mins the wells were emptied and filled with 0.2 M ethanolamine pH 7.5.
7. After 30 mins the wells were washed x3 with TBS Tween20.
8. 100 µl of TBS Tween20 containing 10⁸ plaque forming units of each of the anti-PrP conjugated phage C and phage A was added.
9. After incubation at room temperature for 1 hour the wells were washed x3 with TBS Tween20 and then x2 with TBS.
10. 1 ml of log phase growth XL-1 Blue (Stratagene) E. coli cells were then added to each well and the microtiter plate incubated at 37°C for 60 minutes.
11. The cell suspension from each well was then plated out on a 2xYT (Sigma Aldrich Chemical Company Ltd) 1.5% (w/v) agar containing 100 µg/ml ampicillin and 50 µg/ml chloramphenicol.
12. After incubation overnight at 37°C the number of colonies on each plate were counted.

### Results

The plate derived from the well containing uninfected brain extract contained no colonies of E. coli growth. The plate derived from the well containing infected brain extract showed many colonies of E. coli growth (greater than 1000 colonies). The aggregated PrP was able to link the two phage types such that they could infect the same bacterial host and confer resistance to both antibiotics which were subsequently used for selection in the agar plate. This shows that the method can be used to differentiate the aggregated disease form of a molecule from the unaggregated normal form.

Although the invention has been described with reference to preferred embodiments thereof, the invention is not limited to these embodiments and many modifications may be made within the scope of the claims.

## Claims

1. A method for detecting aggregation or polymerisation of molecules in a sample, which method comprises the steps of:
a) exposing a sample containing the molecules to at least two different labelling moieties each of which will bind to only a single site on each of the said molecules such that binding of one of said labelling moieties to a said site will exclude the binding to the same molecule of a different said labelling moiety; and
b) determining whether at least two different said labelling moieties are present linked together via aggregates or polymers of said molecules.

2. A method as claimed in Claim 1, wherein the said different labelling moieties comprise a first virus and a second virus, the said virus being genetically different, each virus being capable of binding directly or indirectly to only a single site on each of the said molecules to form a bound molecule, such that binding of either one of said first and second viruses to said site excludes the binding of the other of said first and second viruses to said bound molecule, so as to form a virally-bound target material which has a distinctive property when it includes both the first and the second virus linked together via aggregated or polymerised molecules.

3. A method as claimed in Claim 2, wherein step a) comprises exposing the sample simultaneously or sequentially to the first and second viruses, each virus being bound to a monoclonal antibody, under conditions to cause linkage of the monoclonal antibodies to the said sites on the molecules, so as to form a virally-bound target material which has a distinctive property when it include both the first and the second virus linked together via aggregated or polymerised molecules.

4. A method as claimed in Claim 2, wherein the determination step b) comprises the steps of:
c) cultivating, in the presence of the product from step a), an indicator material to which the viruses carried by the virally-bound target material attach so as to cause the indicator material to adopt the distinctive property of the virally-bound target material when the said target material includes both the first and the second virus linked together via aggregated or polymerised molecules; and
d) monitoring the presence or otherwise of indicator material to which both viruses have attached.

5. A method as claimed in Claim 4, wherein the cultivation stage c) is carried out under conditions which indicator material to which both viruses have attached survives but which indicator material to which none or only one of the viruses have attached does not survive.

6. A method as claimed in Claim 2, wherein the viruses are linked to the molecules through an intermediate ligand.

7. A method as claimed in Claim 6, wherein the said intermediate ligand is a monoclonal antibody.

8. A method as claimed in any one of Claims 2 to 7, wherein the first and second viruses encode different genes.

9. A method as claimed in Claim 8, wherein the first virus encodes at least one gene for resistance to a first antibiotic and the second virus encodes at least one gene for resistance to a second different antibiotic, whereby the viruses endow the indicator material with resistance to both antibiotics when the said target material includes both the first and the second virus linked together via aggregated or polymerised molecules.

10. A method as claimed in Claim 4, wherein the indicator material is a bacterial culture.

11. A method as claimed in Claim 10, wherein the cultivation in step c) is carried out in the presence of at least the statistical amount of bacterial cells.

12. A method as claimed in Claim 1, wherein the different labelling moieties respectively comprise a first nucleic acid moiety and a second nucleic acid moiety, each nucleic acid moiety being capable of binding directly or indirectly to only a single site on each of the said molecules to form a bound molecule, such that binding of either one of said first and second nucleic acid moieties to said site excludes the binding of the other of said first and second nucleic acid moieties to said bound molecule, so as to form a nucleic acid-bound target material which has a distinctive property when it includes both the first and the second nucleic acid moieties linked together via aggregated or polymerised molecules.

13. A method as claimed in Claim 12, wherein the nucleic acid moieties are linked to the said molecules via an intermediate ligand.

14. A method as claimed in Claim 13, wherein the intermediate ligand is a monoclonal antibody.

15. A method as claimed in any one of Claims 12 to 14, wherein the nucleic acid moieties comprise oligonucleotides.

16. A method as claimed in Claim 15, wherein the oligonucleotides have from 15 to 25 bases.

17. A method as claimed in any one of Claims 13 to 16, further comprising the step of cleaving the said nucleic acid moieties with a restriction endonuclease that generates short complementary sequences at each end of the nucleic acid moieties prior to coupling to the ligands.

18. A method as claimed in any one of Claims 12 to 17, wherein the determination step b) comprises effecting a ligation reaction of hybridised nucleic acid moieties and then detecting the products of ligation.

19. A method as claimed in any one of the preceding claims, wherein the said molecules are protein molecules.

20. A method of differentiating pathogenic aggregated protein molecules from non-pathogenic, non-aggregated protein molecules, the method comprising the steps of:
a) exposing a sample containing the protein molecules to at least two different labelling moieties each of which will bind to only a single site on each of the said protein molecules such that binding of one of said labelling moieties to a said site will exclude the binding to the same molecule of a different said labelling moiety; and
b) determining whether at least two different said labelling moieties are present linked together via aggregations of said protein molecules.

21. A method as claimed in Claim 20, wherein the said different labelling moieties comprise a first virus and a second virus, each virus being capable of binding directly or indirectly to only a single site on each of the said molecules to form a bound molecule, such that binding of either one of said first and second viruses to said site excludes the binding of the other of said first and second viruses to said bound molecule, so as to form a virally-bound target material which has a distinctive property when it includes both the first and the second virus linked together via aggregated or polymerised molecules; the determination step b) comprising the steps of:
a) cultivating, in the presence of the product from step a), an indicator material to which the viruses carried by the virally-bound target material attach so as to cause the indictor material to adopt the distinctive property of the virally-bound target material when the said target material includes both the first and the second virus linked together via aggregated or polymerised molecules; and
b) monitoring the presence or otherwise of indicator material to which both viruses have attached.

22. A method as claimed in Claim 20, wherein the said different labelling moieties comprise a first nucleic acid moiety and a second nucleic acid moiety, each nucleic acid moiety being capable of binding directly or indirectly to only a single site on each of the said molecules to form a bound molecule, such that binding of either one of said first and second nucleic acid moieties to said site excludes the binding of the other of said first and second nucleic acid moieties to said bound molecule, so as to form a nucleic acid-bound target material which has a distinctive property when it includes both the first and the second nucleic acid moieties linked together via aggregated or polymerised molecules; the determination step b) comprising the steps of:
c) effecting a ligation reaction of hybridised nucleic acid moieties and;
d) detecting the products of ligation.

## Patentansprüche

1. Verfahren zur Detektion einer Aggregation oder Polymerisation von Molekülen in einer Probe, welches Verfahren folgende Schritte umfasst:
a) eine die Moleküle enthaltende Probe wird mindestens zwei unterschiedlichen, markierenden Resten ausgesetzt, von denen jeder nur an einer Stelle eines jeden der Moleküle anbinden wird, so dass eine Bindung von einem der markierenden Reste an einer derartigen Stelle die Bindung mit dem gleichen Molekül eines anderen markierenden Restes ausschließt; und
b) es wird bestimmt, ob mindestens zwei verschiedene der markiereneden Reste über Aggregate oder Polymere der Moleküle miteinander verknüpft vorliegen.

2. Verfahren nach Anspruch 1, wobei die zwei verschiedenen markierenden Reste einen ersten Virus und einen zweiten Virus umfassen, wobei sich die Viren genetisch unterscheiden, jeder Virus direkt oder indirekt sich nur an einer einzigen Stelle an jedem der Moleküle zur Bindung eines gebundenen Moleküls anbinden kann, sodass eine Bindung des ersten oder des zweiten Virus mit dem Ort die Bindung des anderen von dem ersten und dem zweiten Virus mit dem gebundnen Molekül ausschließt, um ein viral gebundenes Targetmaterial zu bilden, das eine unterscheidende Eigenschaft hat, wenn es sowohl den ersten als auch den zweiten Virus umfasst, die über aggregierte oder polymerisierte Moleküle miteinander verknüpft sind.

3. Verfahren nach Anspruch 2, wobei Schritt a) umfasst, dass die Probe gleichzeitig oder nacheinander dem ersten und dem zweiten Virus ausgesetzt wird, wobei jeder Virus an einen monoklonalen Antikörper gebunden ist, und zwar unter Bedingungen, um eine Verknüpfung der monoklonalen Antikörper mit den Stellen an den Molekülen zu bewirken, so dass ein viral gebundenes Targetmaterial gebildet wird, das eine unterscheidende Eigenschaft hat, wenn es sowohl das erste als auch das zweite Virus umfasst, die über aggegierte oder polymerisierte Moleküle verknüpft sind.

4. Verfahren nach Anspruch 2, wo der Bestimmungsschritt b) folgende Schritte umfasst:
c) in der Gegenwart des Produkts aus a) wird ein Indikatormaterials kultiviert, an das sich die von dem viral gebundenen Targetmaterial getragenen Viren anlagern, damit das Indikatormaterial die unterscheidende Eigenschaft des viral gebundenen Targetmaterials annimmt, wenn das Targetmaterial sowohl das erste als auch das zweite Virus umfasst, die über aggegierte oder polymerisierte Moleküle miteinander verbunden
d) ein Vorliegen oder sonstiges von Indikatormaterial wird überwacht, an das sich beide Viren angelagert haben.

5. Verfahren nach Anspruch 4, wobei der Kultivierungsschritt c) unter Bedingungen durchgeführt wird, unter denen das Indikatormaterial, an das sich beide Viren angelagert haben, überlebt, das Indikatormaterial, an welches sich keines oder nur eines der Viren angelagert hat, aber nicht überlebt.

6. Verfahren nach Anspruch 2, wobei die Viren über einen Zwischenliganden mit den Molekühlen verbunden sind.

7. Verfahren nach Anspruch 6, wobei der Zwischenligand ein monoklonaler Antikörper ist.

8. Verfahren nach einem der Ansprüche 2 bis 7, wobei das erste und das zweite Virus unterschiedliche Gene kodieren.

9. Verfahren nach Anspruch 8, wobei das erste Virus mindestens ein Gen zur Resistenz gegenüber einem ersten Antibiotikum kodiert und das zweite Virus mindestens ein Gen zu Resistenz gegenüber einem zweiten, unterschiedlichen Antibiotikum kodiert, wobei die Viren das Indikatormaterial mit Resistenz gegenüber beiden Antibiotika ausstatten, wenn das Targetmaterial sowohl das erste als auch das zweite Virus enthält, die über aggregierte oder polymerisierte Moleküle miteinander verbunden sind.

10. Verfahren nach Anspruch 4, wobei das Indikatormaterial eine Bakterienkultur ist.

11. Verfahren nach Anspruch 10, wobei die Kultivierung in Schritt c) in Gegenwart mindestens der statistischen Menge an Bakterienzellen durchgeführt wird.

12. Verfahren nach Anspruch 1, wobei die unterschiedlichen markierenden Reste jeweils einen ersten Nukleinsäurerest und einen zweiten Nukleinsäurerest umfassen, wobei jeder Nukleinsäurerest sich direkt oder indirekt an nur einer einzigen Stelle an jedem der Moleküle anbinden kann, um ein gebundenes Molekül zu bilden, so dass ein Binden entweder des ersten oder des zweiten Nukleinsäurerests an diese Stelle das Binden des anderen des ersten und des zweiten Nukleinsäurerests an das gebundene Molekül ausschließt, um ein nukleinsäuregebundenes Targetmaterial zu bilden, das eine unterscheidende Eigenschaft hat, wenn es sowohl den ersten als auch den zweiten Nukleinsäurerest umfasst, die über aggregierte oder polymerisierte Moleküle miteinander verbunden sind.

13. Verfahren nach Anspruch 12, wobei die Nukleinsäurereste über einen Zwischenliganden mit den Molekülen verbunden sind.

14. Verfahren nach Anspruch 13, wobei der Zwischenligand ein monoklonaler Antikörper ist.

15. Verfahren nach einem der Ansprüche 12 bis 14, wobei die Nukleinsäurerest Oligonukleotide umfassen.

16. Verfahren nach Anspruch 15, wobei die Oligonukleotide 15 bis 25 Basen haben.

17. Verfahren nach einem der Ansprüche 13 bis 16, des Weiteren umfassend den Schritt eines Spaltens der Nukleinsäurereste mit einer Restriktionsendonuklease, die vor dem Koppeln an die Liganden kurze komplementäre Sequenzen an jedem Ende der Nukleinsäurereste erzeugt.

18. Verfahren nach einem Ansprüche 12 bis 17, wobei der Bestimmungsschritt b) die Durchführung einer Ligationsreaktion der hybridisierten Nukleinsäurereste und ein anschließendes Detektieren der Ligationsprodukte umfasst.

19. Verfahren nach einem der vorstehenden Ansprüche, wobei die Moleküle Proteinmoleküle sind.

20. Verfahren zum Differenzieren von pathogenen, aggregierten Proteinmolekülen gegenüber nicht pathogenen, nicht aggregierten Proteinmolekülen, wobei das Verfahren folgende Schritte umfasst:
a) eine die Proteinmoleküle enthaltende Probe wird mindestens zwei unterschiedlichen, markierenden Resten ausgesetzt, von denen jeder an nur einer einzigen Stelle an jedem der Proteinmoleküle anbindet, so dass eine Bindung einer der kennzeichnenden Reste mit einer Stelle die Bindung des gleichen Moleküls mit einem anderen der kennzeichnenden Reste ausschließt; und
b) es wird bestimmt, ob mindestens zwei unterschiedliche der markierenden Reste vorliegen, die über Aggregationen der Proteinmoleküle miteinander verbunden sind.

21. Verfahren nach Anspruch 20, wobei die unterschiedlichen markierenden Reste einen ersten Virus und einen zweiten Virus umfassen, wobei jeder Virus direkt oder indirekt an nur einer einzigen Stelle an jedem der Moleküle zur Bildung eines gebundenen Moleküls anbinden kann, so dass eine Bindung entweder des ersten oder des zweiten Virus an die Stelle die Bindung des anderen des ersten und zweiten Virus an das gebundene Molekül ausschließt, um ein viral gebundenes Targetmaterial zu bilden, das eine unterscheidende Eigenschaft hat, wenn es sowohl das erste als auch das zweite Virus umfasst, die über aggregierte oder polymerisierte Moleküle miteinander verbunden sind; wobei der Bestimmungsschritt b) folgende Schritte umfasst:
a) in der Gegenwart des Produkts aus Schritt a) wird ein Indikatormaterial kultiviert, an das sich das durch das viral gebundene Targetmaterial getragenen Viren anlagern, damit das Indikatormaterial die unterscheidende Eigenschaft des viral gebundenen Targetmaterials annimmt, wenn das Targetmaterial sowohl das erste als auch das zweite Virus umfasst, die über aggregierte oder polymerisierte Moleküle miteinander verknüpft sind; und
b) ein Vorliegen oder sonstiges von Indikatormaterial wird überwacht, an das sich beide Viren angelagert haben.

22. Verfahren nach Anspruch 20, wobei die unterschiedlichen, markierenden Reste einen ersten Nukleinsäurerest und einen zweiten Nukleinsäurerest umfassen, wobei sich jeder Nukleinsäurerest direkt oder indirekt an nur einer Stelle eines jeden der Molekühle zur Bildung eines gebundenen Moleküls anlagern kann, so dass eine Bindung des ersten oder des zweiten Nukleinsäurerests an dieser Stelle die Bindung des anderen des ersten und zweiten Nukleinsäurerests mit dem gebundenen Molekül ausschließt, um ein nukleinsäuregebundenes Tagetmaterial zu bilden, das eine unterscheidende Eigenschaft hat, wenn es sowohl den ersten und den zweiten Nukleinsäurerest umfasst, die über aggregierte oder polymerisierte Moleküle miteinander verbunden sind; wobei der Bestimmungsschritt b) folgende Schritte umfasst:
c) Auslösen einer Ligationsreaktion von hybridisierten Nukleinsäureresten; und
d) Detektieren der Ligationsprodukte.

## Revendications

1. Procédé pour détecter une agrégation ou une polymérisation de molécules dans un échantillon, lequel procédé comprend les étapes consistant à :
a) exposer un échantillon contenant les molécules à au moins deux fragments de marquage différents, chacun de ces fragments se liera à seulement un site unique sur chacune desdites molécules de sorte que la liaison de l'un desdits fragments de marquage audit site exclura la liaison à la même molécule d'un dit fragment de marquage différent; et
b) déterminer si au moins deux desdits fragments de marquage différents sont présents, liés ensembles via des agrégats ou des polymères desdites molécules.

2. Procédé selon la revendication 1, dans lequel lesdits fragments de marquage différents comprennent un premier virus et un second virus, ledit virus étant génétiquement différent, chaque virus étant capable de se lier directement ou indirectement à seulement un site unique sur chacune desdites molécules pour former une molécule liée, de sorte que la liaison de l'un ou l'autre desdits premier et second virus audit site exclut la liaison de l'autre desdits premier et second virus à ladite molécule liée, de façon à former une matière-cible liée au virus ayant une propriété distinctive lorsqu'elle inclut à la fois le premier et le second virus liés ensembles via des molécules agrégées ou polymérisées.

3. Procédé selon la revendication 2, dans lequel l'étape a) comprend l'exposition de l'échantillon simultanément ou séquentiellement au premier et second virus, chaque virus étant lié à un anticorps monoclonal, dans des conditions qui entraînent la liaison des anticorps monoclonaux aux-dits sites sur les molécules, de sorte à former une matière-cible liée au virus ayant une propriété distinctive lorsqu'elle inclut à la fois le premier et le second virus liés ensembles via des molécules agrégées ou polymérisées.

4. Procédé selon la revendication 2, dans lequel la détermination de l'étape b) comprend les étapes consistant à :
c) cultiver, en présence du produit de l'étape a), un matériel indicateur auquel les virus portés par la matière-cible liée au virus s'attachent de façon à entraîner le matériel indicateur à adopter la propriété distinctive de la matière-cible liée au virus lorsque ladite matière cible inclut à la fois le premier et le second virus liés ensembles via des molécules agrégées ou polymérisées ; et
d) contrôler la présence ou non du matériel indicateur auquel les deux virus se sont attachés.

5. Procédé selon la revendication 4, dans lequel la culture à l'étape c) est réalisée dans des conditions dans lesquelles le matériel indicateur survit lorsque les deux virus y sont attachés, mais dans lesquelles le matériel indicateur ne survit pas lorsqu'aucun virus ou seulement un des virus s'y est attaché.

6. Procédé selon la revendication 2, dans lequel les virus sont liés aux molécules par un ligand intermédiaire.

7. Procédé selon la revendication 6, dans lequel ledit ligand intermédiaire est un anticorps monoclonal.

8. Procédé selon l'une des revendications 2 à 7, dans lequel les premier et second virus codent pour des gènes différents.

9. Procédé selon la revendication 8, dans lequel le premier virus code pour au moins un gène de résistance à un premier antibiotique et le second virus code pour au moins un gène de résistance à un second antibiotique différent, grâce à quoi les virus dotent le matériel indicateur d'une résistance aux deux antibiotiques lorsque ladite matière cible inclut à la fois le premier et le second virus liés ensembles via des molécules agrégées ou polymérisées.

10. Procédé selon la revendication 4, dans lequel le matériel indicateur est une culture bactérienne.

11. Procédé selon la revendication 10, dans lequel la culture de l'étape c) est réalisée en présence d'au moins une quantité statistique de cellules bactériennes.

12. Procédé selon la revendication 1, dans lequel les fragments de marquage différents comprennent respectivement un premier fragment d'acide nucléique et un second fragment d'acide nucléique, chaque fragment d'acide nucléique étant capable de se lier directement ou indirectement à seulement un site unique sur chacune desdites molécules pour former une molécule liée, de sorte que la liaison de l'un ou l'autre desdits premier et second fragments d'acide nucléique audit site exclut la liaison de l'autre desdits premier et second fragments d'acide nucléique à ladite molécule liée, de façon à former une matière-cible liée à un acide nucléique qui a une propriété distinctive lorsqu'elle inclut à la fois le premier et le second fragments d'acide nucléique liés ensemble via des molécules agrégées ou polymérisées.

13. Procédé selon la revendication 12, dans lequel les fragments d'acide nucléique sont liés aux-dites molécules via un ligand intermédiaire.

14. Procédé selon la revendication 13, dans lequel le ligand intermédiaire est un anticorps monoclonal.

15. Procédé selon l'une des revendications 12 à 14, dans lequel les fragments d'acide nucléique comprennent des oligonucléotides.

16. Procédé selon la revendication 15, dans lequel les oligonucléotides ont de 15 à 25 bases.

17. Procédé selon l'une des revendications 13 à 16, comprenant en outre l'étape de clivage desdits fragments d'acide nucléique avec une endonucléase de restriction qui génère des séquence complémentaires courtes à chaque extrémité des fragments d'acide nucléique avant le couplage aux ligands.

18. Procédé selon l'une des revendications 12 à 17, dans lequel la détermination de l'étape b) comprend la mise en oeuvre d'une réaction de ligature des fragments d'acide nucléique hybridés puis la détection des produits de ligature.

19. Procédé selon l'une des revendications précédentes, dans lequel lesdites molécules sont des molécules protéiques.

20. Procédé de différenciation de molécules protéiques agrégées pathogènes de molécules protéiques non agrégées non pathogènes, le procédé comprenant les étapes consistant à :
a) exposer un échantillon contenant les molécules protéiques à au moins deux fragments de marquage différents, chacun de ces fragments se liera à seulement un site unique sur chacune desdites molécules protéiques de sorte que la liaison de l'un desdits fragments de marquage audit site exclura la liaison à la même molécule d'un dit fragment de marquage différent; et
b) déterminer si au moins deux desdits fragments de marquage différents sont présents, liés ensembles via des agrégations desdites molécules protéiques.

21. Procédé selon la revendication 20, dans lequel lesdits fragments de marquage différents comprennent un premier virus et un second virus, chaque virus étant capable de se lier directement ou indirectement à seulement un site unique sur chacune desdites molécules pour former une molécule liée, de sorte que la liaison de l'un ou l'autre desdits premier et second virus audit site exclut la liaison de l'autre desdits premier et second virus à ladite molécule liée, de façon à former une matière-cible liée au virus ayant une propriété distinctive lorsqu'elle inclut à la fois le premier et le second virus liés ensembles via des molécules agrégées ou polymérisées ; la détermination de l'étape b) comprenant les étapes consistant à :
a) cultiver, en présence du produit de l'étape a), un matériel indicateur auquel les virus portés par la matière-cible liée au virus s'attachent de façon à entraîner le matériel indicateur à adopter la propriété distinctive de la matière-cible liée au virus lorsque ladite matière cible inclut à la fois le premier et le second virus liés ensembles via des molécules agrégées ou polymérisées ; et
b) contrôler la présence ou non du matériel indicateur auquel les deux virus se sont attachés.

22. Procédé selon la revendication 20, dans lequel lesdits fragments de marquage différents comprennent un premier fragment d'acide nucléique et un second fragment d'acide nucléique, chaque fragment d'acide nucléique étant capable de se lier directement ou indirectement à seulement un site unique sur chacune desdites molécules pour former une molécule liée, de sorte que la liaison de l'un desdits premier et second fragments d'acide nucléique audit site exclut la liaison de l'autre desdits premier et second fragment d'acide nucléique à ladite molécule liée, de façon à former une matière-cible liée à un acide nucléique ayant une propriété distinctive lorsqu'elle inclut à la fois le premier et le second fragments d'acide nucléique liés ensembles via des molécules agrégées ou polymérisées ; la détermination de l'étape b) comprenant les étapes consistant à :
c) réaliser une réaction de ligature des fragments d'acide nucléique hybridés et ;
d) détecter les produits de ligature.
